# EUROPEAN PATENT SPECIFICATION

(11) **EP 1 543 861 B1**
(45) Date of publication and mention of the grant of the patent: **14.03.2007**
(21) Application number: 04257963.1
(22) Date of filing: 20.12.2004
(51) Int. Cl.: A61M 39/02

(54) **Subcutaneous injection port for applied fasteners**
Subkutane Zugangsöffnung
Port d'accès sous-cutané

(30) Priority: 19.12.2003 US 741127
(43) Date of publication of application: 22.06.2005
(73) Proprietor: ETHICON ENDO-SURGERY, INC., Cincinnati, Ohio 45242 (US)
(72) Inventor: Conlon, Sean P., Loveland Ohio 45140 (US); Hunt, John V., Cincinnatti Ohio 45241 (US)
(74) Representative: Tunstall, Christopher Stephen

(56) References cited:
- DE-A1- 19 751 791
- US-A- 5 352 204

## Description

### TECHNICAL FIELD

The present invention relates in general to surgically implantable fluid injection ports, and is particularly directed to fasteners and methods for fastening subcutaneous peripherally attached ports. The invention will be specifically disclosed in connection with injection ports used with adjustable gastric bands, although the fasteners of the present invention may be used with many different subcutaneuosly attached devices, including injection ports used for vascular access such as the infusion of medications and blood draws.

### Background Of The Invention

Injection ports are placed beneath the skin of a body for injecting fluids into the body, such as for infusing medication, blood draws, and many other applications, including adjustable gastric bands. Since the early 1980s, adjustable gastric bands have provided an effective alternative to gastric bypass and other irreversible surgical weight loss treatments for the morbidly obese. The gastric band is wrapped around an upper portion of the patient's stomach, forming a stoma that restricts food passing from an upper portion to a lower portion of the stomach. When the stoma is of the appropriate size, food held in the upper portion of the stomach provides a feeling of fullness that discourages overeating. However, initial maladjustment or a change in the stomach over time may lead to a stoma of an inappropriate size, warranting an adjustment of the gastric band. Otherwise, the patient may suffer vomiting attacks and discomfort when the stoma is too small to reasonably pass food. At the other extreme, the stoma may be too large and thus fail to slow food moving from the upper portion of the stomach, defeating the purpose altogether for the gastric band.

In addition to a latched position to set the outer diameter of the gastric band, adjustability of gastric bands is generally achieved with an inwardly directed inflatable balloon, similar to a blood pressure cuff into which fluid, such as saline, is injected through a fluid injection port to achieve a desired diameter. Since adjustable gastric bands may remain in the patient for long periods of time, the fluid injection port is typically installed subcutaneously to avoid infection, for instance in front of the sternum. Adjusting the amount of fluid in the adjustable gastric band is achieved by inserting a Huber needle through the skin into a silicon septum of the injection port. Once the needle is removed, the septum seals against the hole by virtue of compressive load generated by the septum. A flexible conduit communicates between the injection port and the adjustable gastric band.

The traditional surgical technique for securing a fluid injection port developed for vascular uses has been applying sutures through a series of holes spaced about a peripheral base flange. While generally effective, suturing often proves to be difficult and time consuming, even more so with adjustable gastric band which are intended for the morbidly obese. A significant thickness of fat tissue may underlie the skin, causing difficulties as the surgeon attempts to apply sutures to deeply recessed tissues (e.g., 5-10 cm) to secure the port, often requiring 10-15 minutes to complete. Further, if the injection port is not sutured properly, the port may flip over, making subsequent injections difficult or impossible.

Recently, a surgical stapler has been described in a German patent application No. 197 51 791.9 to Pier wherein a hat-shaped injection port includes tangentially aligned linear grooves spaced around its perimeter. A pair of holes in each linear groove receives a traditional bent wire staple. A stapler holds the staples in place and has downwardly moving forming member that presses upon the shoulders of each staple. Due to the position of the holes in the linear groove, pointed ends of the staple are deformed inwardly to grip underlying tissue.

This Pier stapler thus attaches the staples through a deformation that may prove difficult to release should the initial placement be undesirable or removal of the gastric band be appropriate. Further, because the device must permanently deform a multiplicity of stainless steel or titanium staples, a more complicated mechanism is required to provide the surgeon with enough mechanical advantage to form the staples easily. The Pier injection port also requires a custom stapler handle that is not useful for any other purpose, which may be an undesirable expense for surgeons who do not perform numerous placements of the Pier injection port.

US 5,352,204 discloses an implantable patient access port which incorporates an internal reservoir and a means for refilling the reservoir. The port comprises a housing which forms a mounting surface for supporting the device subcutaneously. Support is provided by a radially extending flange at the base of the housing which is provided with mounting holes for accepting sutures or surgical staples to mount the housing to tissue.

While a custom stapler for an injection port may have advantages over suturing, such as the time required to accomplish the attachment, it is believed that other approaches to attaching an injection port may have additional advantages or features not provided by traditional suturing or a stapler using bent wire staples. Consequently, a significant need exists for a fluid injection port suitable for subcutaneous attachment that is quickly attachable yet is secure over a long period of time.

### Brief Summary Of The Invention

As described herein, there is provided an injection port for injecting fluids into a body, said port comprising: a frustoconical housing for placement beneath the skin of the body, said housing including means for receiving a needle; and a plurality of circumferentially spaced recesses formed through said frustoconical housing for receiving at least one fastener and aligning said at least one fastener in its proper position for deployment in tissue adjacent said housing. The injection port may also include a guidance configuration adapted to receive and guide a delivery end of a device for implanting fasteners in a position to retain the housing.

### Brief Description Of The Drawings

The accompanying drawings incorporated in and forming a part of the specification illustrate several aspects of the present invention, and together with the description serve to explain the principles of the invention. In the drawings:

FIG. 1 is a diagrammatic drawing showing an injection port constructed in accordance with the present invention, connected to an adjustable gastric band wrapped around an upper part of a stomach.

FIG. 2 is a perspective view of the injection port shown in Fig. 1.

FIG. 2A is a fragmentary, enlarged side view taken along line 2A-2A of FIG. 2.

FIG. 3 is a perspective view of the injection port of FIG. 1 during attachment to a fascia layer using an existing surgical instrument.

FIG. 4 is a fragmentary, enlarged side view of the injection port and surgical instrument as shown in FIG. 3.

FIG. 5 is a perspective view showing a fastener securing an injection port in place.

FIG. 6 is a side view of the fastener shown in FIG. 5.

FIG. 7 is a cross sectional drawing of the transfer tube of the surgical instrument with fasteners.

Reference will now be made in detail to the present preferred embodiment of the invention, an example of which is illustrated in the accompanying drawings.

### Detailed Description Of An Embodiment Of The Present Invention

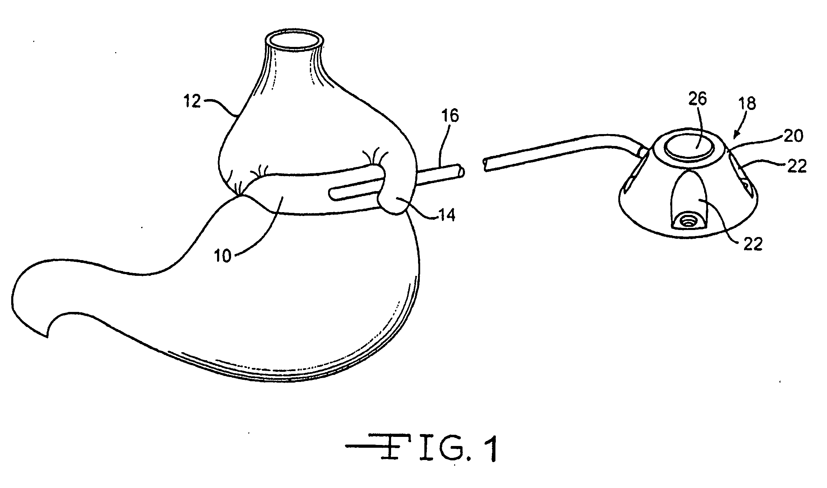

Referring now to the drawings in detail, wherein like numerals indicate the same elements throughout the views, FIG. 1, adjustable gastric band 10 is shown wrapped around an upper portion of stomach 12, kept in place by attaching the two ends together and extending portion 14 of the stomach 12 over adjustable gastric band 10 by suturing portion 14 to the stomach. One end of flexible conduit 16 is in fluid communication with the internal cavity of the balloon (not shown), with the other end being in fluid communication with an internal cavity (not shown) of injection port 18. At the time adjustable gastric band 10 is implanted around a portion of the stomach, remote injection port 18 is also implanted at a suitable location, usually within the rectus sheaths, for transcutaneous access via a Huber needle.

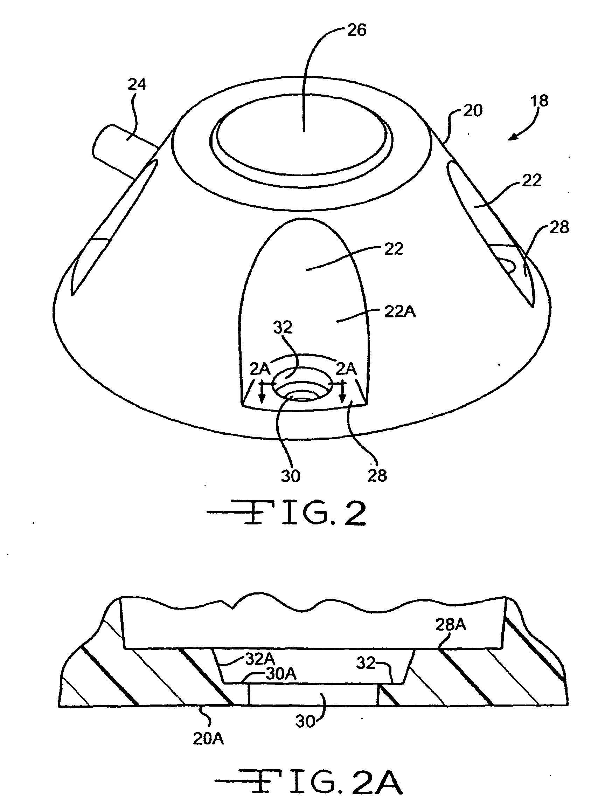

Referring also to FIGS. 2 and 2A, injection port 18 includes frustroconical housing 20 having four circumferentially spaced recesses 22 formed therein. Nipple 24 is in fluid communication with the internal cavity defined by housing 20, to which flexible conduit 16 is attached at some point in the procedure, typically after injection port 18 has been implanted. Fluid is added to or removed from the interior cavity of injection port 18 by inserting a Huber needle percutaneously into silicone septum 26 of the injection port 18. Although septum 26 is made of silicon, the means of the injection port for receiving a needle includes any structure configured to self seal after puncture with a non-coring needle.

Although a specific configuration for injection port 18 is disclosed herein, there are many suitable configurations which may be used in conjunction with the present invention. For example, injection port 18 may include an annular flange about its base instead of recesses 22, with the fastener openings and guidance configuration formed in the annular flange.

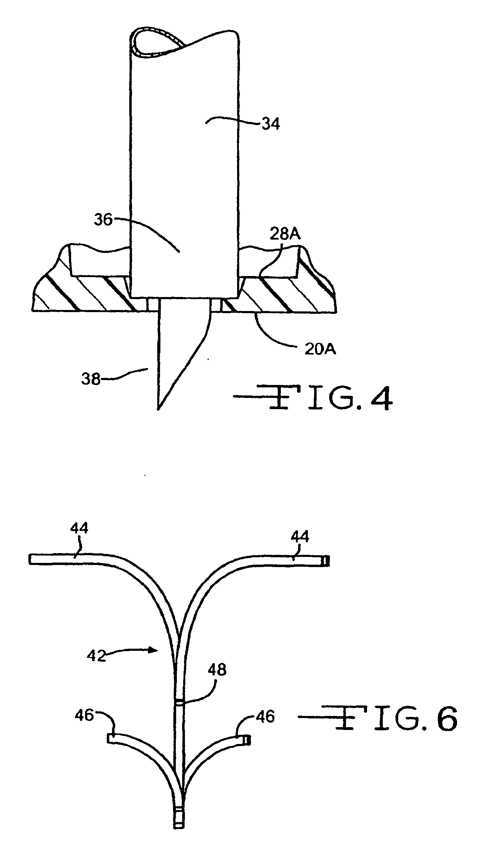

Each recess 22 includes side wall 22a, and base 28 which has upper surface 28a which is generally parallel to distal end 20a of housing 20 (see FIG. 4). Each base 28 includes a respective fastener opening 30 and includes a device guidance configuration which is adapted to receive and guide the delivery end of a device for implanting fasteners so to deliver fasteners into fastener opening 30 in the proper position and depth. In the depicted embodiment, the device guidance configuration is configured as device guidance bores 32 which is adapted to guide and receive a device having a cylindrical delivery end, and is shown as a counter-bore formed into surface 28a, axially aligned with fastener opening 30. In the embodiment depicted, guidance bore 32 includes frustroconical wall 32a, which has a larger diameter at surface 28a, leading to a smaller diameter at surface 30b. The diameter of bore 32 at surface 28a is larger than the diameter end which assists to locate initially the delivery end in bore 32, with wall 32a guiding the advancement of the delivery end into the proper position at surface 30a. The diameter of bore 32 at surface 30a maintains the delivery end in the proper position relative to fastener opening 30 providing enough clearance so as not to bind and not to require excessive force to seat the delivery end in bore 32 against surface 30a. Guidance bore 32 and wall 32a are not limited to frustroconical or inclined shapes. Wall 32a may, for example, be perpendicular to surface 30a, adequately dimensioned to allow the delivery end into the proper position for implanting the fastener.

Since there are a variety of end configurations for delivery devices, the device guidance configuration may have a variety of configurations, as long as it has a shape which is complementary to and will cooperate with the particular end configuration. A single shape such as device guidance bore 32 as illustrated may cooperate with a variety of end configurations for delivery devices. The device guidance configuration may alternatively include raised portions extending above surface 28a to receive and guide the delivery end.

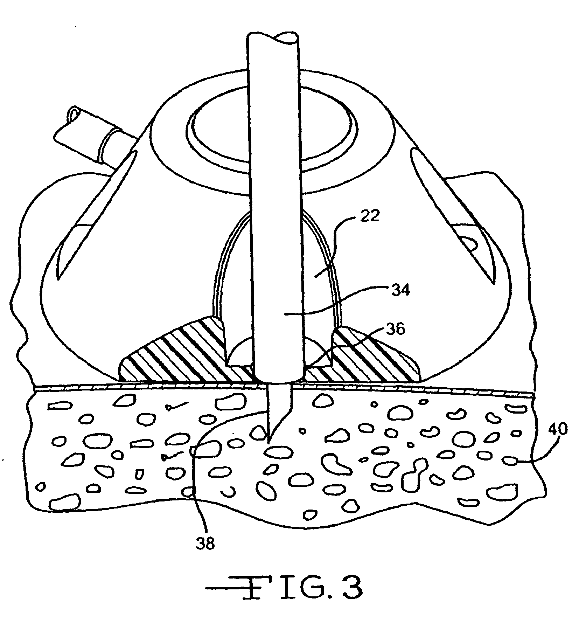

Referring to FIG. 3, there is shown surgical device 34 which is configured to implant fasteners. A suitable device is described in United States Patent No. 6,447,524. Recesses 22 provide adequate clearance for device 34, and for the fasteners delivered therefrom. Device 34 includes delivery end 36, which as shown in FIG. 3, has been guided by bore 32 into the proper axially aligned position with fastener opening 30. When device 34 is actuated, effector 38 extends from delivery end 36 for the placement and release of a fastener, extending through fastener opening 30 and into fascia layer 40, creating the path for the fastener. The fastener is delivered into fascia layer 40 and engages upper surface 28a as effector 38 is withdrawn into device 34 and device 34 is withdrawn from bore 32.

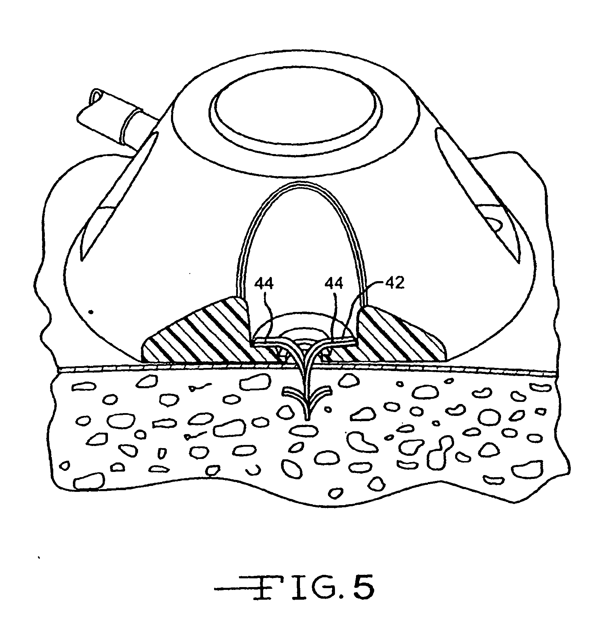

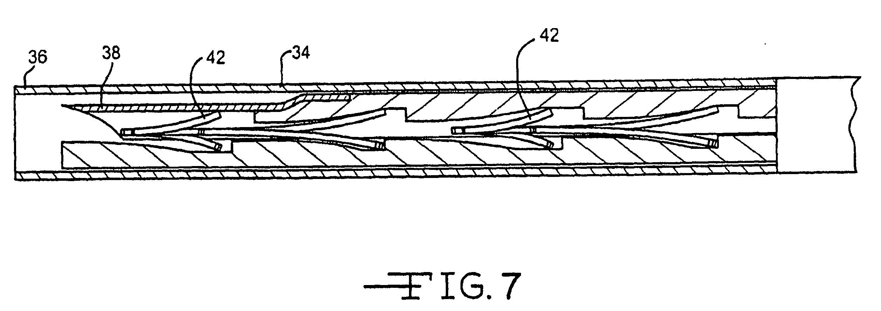

Referring to FIG. 5, fastener 42 is shown in its proper deployed position, aligned and positioned by side wall 22a, base 28 and opening 30, thereby securing injection port 18 in a deployed/implanted position. Referring also to FIG. 6, fastener 42 includes port hold down legs 44, tissue retention legs or barbs 46, and continuous body member 48 which connect port hold down legs 44 and retention legs 46 together. Port hold down legs 44 extend in opposite directions and engage upper surface 28a.

Fastener 44 is constructed essentially the same as the fastener of United States Patent No. 6,447,524, except that port hold down legs 44 are generally planar, allowing generally flat engagement with upper surface 28a, such that retention legs 46 extend further to an appropriate depth into fascia layer 40. The thickness of base 28 is selected so that retention legs 46 penetrate to a depth that will assure proper fixation of injection port 18. Legs 44 being flat allows more of fastener 42 to extend below distal end bottom 20a than would a fastener of same length if the port hold down legs were arcuate as shown in United States Patent No. 6,447,524. This allows the overall length of fastener 42 to be the same as the fastener shown in United States Patent No. 6,447,524, thereby allowing fastener 42 to work in the surgical disclosed in United States Patent No. 6,447,524, as shown in FIG 7.

Practice of the present invention is not limited to the specific delivery device and fastener disclosed herein. Other fastener delivery devices and other fasteners, whether or not currently commercially available, may be used with an injection port constructed in accordance with the present invention, with the guidance configuration adapted to receive and guide the delivery end of such devices, and providing adequate clearance for the device and fastener.

For example, guidance configuration 32 may be adapted to receive and guide the delivery end of the stapler described in United States Patents Numbers 5,634,584, 5,829,662, 5,588,581, 5,381,943. For use with such staplers, or any other fastener delivery devices, guidance configuration 32 is shaped complementary to a sufficient number of physical features of the delivery end of the stapler to receive and guide the delivery end adequately. Guidance configuration 32 may orient the staples tangentially, in which two openings in the base per staple are required, or may orient the staples outwardly, such as radially outward, using only a single opening in the base.

Thus, although the delivery device guidance configuration of the present invention has been described herein as being part of base 28 adjacent fastener opening 30, such guidance may be formed in various configurations and locations suited to the particular delivery device utilized.

In summary, numerous benefits have been described which result from employing the concepts of the invention. The foregoing description of one or more embodiments of the invention has been presented for purposes of illustration and description. It is not intended to be exhaustive or to limit the invention to the precise form disclosed. Obvious modifications or variations are possible in light of the above teachings. The one or more embodiments were chosen and described in order to best illustrate the principles of the invention and its practical application to thereby enable one of ordinary skill in the art to best utilize the invention in various embodiments and with various modifications as are suited to the particular use contemplated. It is intended that the scope of the invention be defined by the claims appended hereto.

## Claims

1. An injection port (18) for injecting fluids into a body, said port (18) comprising:
a. a frustoconical housing (20) for placement beneath the skin of the body, said housing (20) including means for receiving a needle; and
b. a plurality of circumferentially spaced recesses (22) formed through said frustoconical housing (20), wherein each recess (22) includes a base (28), and a respective opening (30) formed through said base, said recesses (22) for receiving at least one fastener and aligning said at least one fastener in its proper position for deployment in tissue adjacent said housing (20).

2. The port of claim 1, wherein each recess (22) extends proximally above a distal end of said housing.

3. The port (18) of claim 2, wherein each said recess comprises a side wall (22a).

4. The port (18) of claim 1, wherein said plurality of recesses comprise means for receiving and guiding a delivery end of a device for implanting said at least one fastener in the tissue.

5. The port (18) of claim 4, wherein each recess (22) includes a base (28), and a respective opening (30) formed through said base (28), and each said recess comprises a respective guidance configuration in each respective base aligned with said opening adapted to receive and guide the delivery end.

6. The port of claim 5, wherein said guidance configuration comprises a counter bore.

## Patentansprüche

1. Injektions-Anschlußeinrichtung (18) zum Injizieren von Fluiden in einen Körper, wobei die Anschlußeinrichtung (18) aufweist:
a. ein kegelstumpfartiges Gehäuse (20) für die Anordnung unterhalb der Haut des Körpers, wobei das Gehäuse (20) eine Einrichtung zum Aufnehmen einer Nadel umfaßt; und
b. eine Vielzahl am Umfang beabstandeter Ausnehmungen (22), die durch das kegelstumpfartige Gehäuse (20) gebildet sind, wobei jede Ausnehmung (22) eine Basis (28) und eine jeweilige Öffnung (30), die durch die Basis gebildet ist, umfaßt, wobei die Ausnehmungen (22) zum Aufnehmen wenigstens eines Befestigungselementes und zum Ausrichten des wenigstens einen Befestigungselementes in seiner richtigen Position für den Einsatz in Gewebe angrenzend an das Gehäuse dienen.

2. Anschlußeinrichtung nach Anspruch 1, bei der jede Ausnehmung (22) sich proximal oberhalb eines distalen Endes des Gehäuses erstreckt.

3. Anschlußeinrichtung (18) nach Anspruch 2, bei der jede Ausnehmung eine Seitenwand (22a) aufweist.

4. Anschlußeinrichtung (18) nach Anspruch 1, bei der die Vielzahl von Ausnehmungen eine Einrichtung zum Aufnehmen und Führen eines Abgabeendes einer Vorrichtung zum Implantieren des wenigstens einen Befestigungselementes in dem Gewebe aufweist.

5. Anschlußeinrichtung (18) nach Anspruch 4, bei der jede Ausnehmung (22) eine Basis (28) und eine jeweilige Öffnung (30), die durch die Basis (28) gebildet ist, umfaßt und jede Ausnehmung eine jeweilige Führungsbauform in jeder jeweiligen Basis aufweist, die mit der Öffnung ausgerichtet ist, um das Abgabeende aufzunehmen und zu führen.

6. Anschlußeinrichtung nach Anspruch 5, bei dem die Führungsbauform eine Senkbohrung aufweist.

## Revendications

1. Port d'injection (18) pour injecter des fluides dans un corps, ledit port (18) comprenant :
a. une enveloppe tronconique (20) à placer en dessous de la peau du corps, ladite enveloppe (20) comprenant des moyens pour recevoir une aiguille ; et
b. une pluralité d'évidements circonférentiellement espacés (22) formés à travers ladite enveloppe tronconique (20), dans lequel chaque évidement (22) comprend une base (28) et une ouverture respective (30) formée à travers ladite base, lesdits évidements (22) recevant au moins une attache et alignant ladite au moins une attache dans sa position correcte pour la déployer dans un tissu à proximité de ladite enveloppe (20).

2. Port selon la revendication 1, dans lequel chaque évidement (22) s'étend de façon proximale au-dessus d'une extrémité distale de ladite enveloppe.

3. Port (18) selon la revendication 2, dans lequel chacun desdits évidements comprend une paroi latérale (22a).

4. Port (18) selon la revendication 1, dans lequel ladite pluralité d'évidements comprend des moyens pour recevoir et guider une extrémité de délivrance d'un dispositif pour implanter ladite au moins une attache dans le tissu.

5. Port (18) selon la revendication 4, dans lequel chaque évidement (22) comprend une base (28) et une ouverture respective (30) formée à travers ladite base (28), et chacun desdits évidements comprend une configuration de guidage respective dans chaque base respective alignée avec ladite ouverture adaptée pour recevoir et guider l'extrémité de délivrance.

6. Port selon la revendication 5, dans lequel ladite configuration de guidage comprend un lamage.
